# EUROPEAN PATENT SPECIFICATION

(11) **EP 1 496 758 B1**
(45) Date of publication and mention of the grant of the patent: **03.09.2008**
(21) Application number: 03718451.2
(22) Date of filing: 18.04.2003
(51) Int. Cl.: A23L 1/305, A23L 1/30, A23C 9/152, A23L 1/308, A61K 38/17, A61K 31/201, A61K 31/20

(54) **COMPOSITIONS COMPRISING MILK PROTEIN CONCENTRATE AND FATTY ACID AND PROCESSES OF THEIR PREPARATION**
ZUSAMMENSETZUNGEN ENTHALTEND MILCHPROTEINKONZENTRAT UND FETTSÄURE UND VERFAHREN ZUR HERSTELLUNG
COMPOSITIONS COMPRENANT UN CONCENTRE DE PROTEINE DE LAIT ET UN ACIDE GRAS ET PROCEDES DE PREPARATION

(30) Priority: 24.04.2002 US 375653 P
(43) Date of publication of application: 19.01.2005
(73) Proprietor: THE PROCTER & GAMBLE COMPANY, Cincinnati, Ohio 45202 (US)
(72) Inventor: COOK, Lisa, Ann, Mason, OH 45040 (US)
(74) Representative: Clemo, Nicholas Graham
(86) International application number: PCT/US2003/012048
(87) International publication number: WO 2003/090559

(56) References cited:
- WO-A-02/00042
- WO-A-98/21953
- GB-A- 1 464 571
- US-A- 4 298 601
- US-A- 5 700 782
- US-A1- 2002 019 334
- US-A1- 2002 044 988
- US-B1- 6 207 638

## Description

### FIELD OF THE INVENTION

The present invention relates to compositions comprising milk protein and a fatty acid material. The compositions are particularly useful as food or beverage compositions.

### BACKGROUND OF THE INVENTION

The use of fatty acids, particularly in orally administered compositions, is beneficial for a variety of health concerns. For example, WO 02/00042, Jandacek et al., published January 3, 2002, describes the use of fatty acids for body weight management. The described approach to body weight management includes orally administered compositions containing a fatty acid, which induces satiety in mammals, thereby reducing food consumption. Other uses of fatty acids have been recently promoted, including the use of omega-3-fatty acids in various compositions for the improvement in, for example, cardiac and skin health. US 2002/019334 discloses a nutritional intervention composition for enhancing and extending satiety, containing GMP or CMP, a long chain fatty acid, casein, whey or soy and fibre, which is in the form of a dry powder and may be added to a beverage.

Unfortunately, however, the formulation of such fatty acids in compositions that are acceptable to consumers is not a trivial matter. Fatty acids are susceptible to instability, including degradation, resulting in rancidity. Such instability not only affects to the flavor profile of the fatty acid, but can affect the deliverable health benefit as well. Moreover, even wherein the fatty acid remains stable, the fatty acid is often undesirable from a flavor perspective. A primary example of this undesirability includes omega-3-fatty acids, which can exhibit a fishy off-flavor. It would therefore be desirable to provide compositions that alleviate the problems associated with fatty acid instability and / or undesirable flavor profile.

Various components are known to provide some level of flavor masking in orally administered compositions. For example, inclusion of strong flavors can mask undesirable flavors to a limited extent. However, these strong flavors are often by themselves undesirable, depending upon consumer preference or the type of composition that is desired, and may not alleviate issues associated with instability.

Milk protein has been used in combination with fatty acids which are in the form of glyceryl esters, as with fat. Surprisingly, the present inventor has discovered that milk protein, which is at least partially delivered via milk protein concentrate specifically, provides excellent fatty acid stabilization, particularly physical stabilization, and flavor masking benefits. As a particular unexpected result, the present inventor has discovered that compositions that do not utilize the milk protein concentrate, but utilize only milk, dry milk, free caseinates, or soy may not provide the same stabilization or flavor masking. Without intending to be limited by theory, it is believed that milk protein concentrate is particularly useful for the stabilization of fatty acids due to a variety of reasons. For example, it is believed that the phospholipids that are present in the milk protein concentrate provide important benefits with respect to the stabilization or flavor masking of fatty acids. Alternatively or additionally, again without being bound by theory, certain components ordinarily found in higher levels in milk or dry milk, for example, lactose or various salts, may compromise stabilization or flavor masking of the fatty acid. To illustrate, salts may hinder physical stability of a given composition due to electrostatic charge interactions with the fatty acid or protein components. By reducing salt content, as with typical milk protein concentrate relative to milk or dry milk, such negative electrostatic charge interactions may be decreased or even eliminated.

The inventor has discovered that utilization of milk protein concentrate is therefore highly desirable for use herein. The resulting compositions of the present invention therefore enable the use of fatty acid material in milk protein compositions in a manner that is satisfactory to the consumer.

### SUMMARY OF THE INVENTION

The present invention relates to compositions comprising:
(a) from 0.5% to 4% milk protein concentrate ; wherein the milk protein concentrate comprises at least 65% milk protein; and
(b) less than 6% by milk
(c) a fatty acid material selected from fatty acids, non-glyceryl esters thereof, and mixtures thereof;
wherein when the composition comprises less than 0,027, ethyl oleate, oleic acid, and mixtures thereof, then the composition also comprises less that 1% lactose from second ethes than the mile protein cencentrate.

All compositions of the present invention are particularly useful as food or beverage compositions.

### DETAILED DESCRIPTION OF THE INVENTION

All percentages and ratios are calculated by weight unless otherwise indicated. All percentages and ratios are calculated based on the total composition unless otherwise indicated.

Referenced herein are trade names for components including various ingredients utilized in the present invention. The inventor herein does not intend to be limited by materials under a certain trade name. Equivalent materials (*e*.*g*., those obtained from a different source under a different name or reference number) to those referenced by trade name may be substituted and utilized in the descriptions herein.

In the description of the invention various embodiments or individual features are disclosed. As will be apparent to the ordinarily skilled practitioner, all combinations of such embodiments and features are possible and can result in preferred executions of the present invention.

The compositions herein may comprise, consist essentially of, or consist of any of the elements as described herein.

As will be also be apparent, all combinations of the embodiments and features taught in the foregoing disclosure are possible and can result in preferred executions of the invention.

### The Compositions of the Present Invention

The present compositions are useful for a variety of purposes, particularly as foods or beverages. In particular, without intending to be limited by theory, it is believed that the present compositions exhibit excellent fatty acid stabilization and flavor masking properties due to the milk protein concentrate used therein.

In an optional embodiment, all compositions may less than 5%, preferably less than 3%, even more preferably less than 1%, and most preferably less than 0.5% of lactose, all by weight of the composition.

The various components of the present compositions are described further as follows:

### Milk Protein

The present compositions comprise milk protein. The milk protein present in the compositions herein are important for the stabilization or flavor masking of the fatty acid material. Moreover, unexpectedly, the inventor has found that the particular source of the milk protein is important for optimization of these properties. In particular, the milk protein present in the compositions should be at least partially derived from milk protein concentrate. In preparation of the compositions, therefore, milk protein concentrate should be combined with the other components of the composition.

Milk protein concentrate is commonly understood in the art and may be obtained from a variety of commercial sources or otherwise prepared from milk or other milk source. The milk protein concentrate can comprise, for example, intact milk protein, milk protein hydrosylate, or any combination thereof. Milk protein concentrate is prepared via milk ultrafiltration or other means such that the lactose or salt content is reduced, thereby enhancing the protein content. In contrast, in dry and condensed milk, water is removed but all other components of milk are substantially maintained. Without intending to be limited by theory, it is believed that the relative concentration of phospholipids in the milk protein concentrate, or lactose or salt reduction within milk protein concentrate, may be important for the optimal benefits delivered by the compositions herein.

Milk proteins, within milk protein concentrate, are primarily caseins and whey proteins (the ratio of caseins to whey proteins in milk protein concentrate is typically about 80:20, by weight). The milk protein concentrate utilized herein comprises at least 65%, more preferably at least about 70%, and most preferably at least about 75% protein, by weight of the milk protein concentrate.

The compositions herein comprise from 0.5% to 4%, and preferably from 1% to 3.5% milk protein concentrate, all by weight of the composition. Most preferably, each of these foregoing amounts is based on milk protein concentrate comprising at least about 80% protein, by weight of the milk protein concentrate. Amounts of milk protein concentrate can therefore be appropriately adjusted wherein the concentrate contains more or less protein content.

The milk protein herein is at least partially derived from milk protein concentrate. As such, the compositions may also comprise other sources of milk protein, such as fluid milk (whether whole, skim, or otherwise) or dry milk (whether skim or otherwise), or a variety of other sources. In a preferred embodiment of the present invention, in addition to partial derivation from milk protein concentrate, the milk protein is additionally derived from dry skim milk. Stability and flavor profiles exhibited by the present compositions may not be compromised through use of such sources, since a reduced amount of such sources will be typically used as a result of derivation of other milk protein from the milk protein concentrate.

For example, the milk protein of the present compositions may also be at least partially derived from dry milk (preferably dry skim milk). When milk protein is partially derived from such dry milk, the compositions comprise less than 6% dry milk, all by weight of the composition. A typical composition of the present composition will comprise from 4% to 6% dry milk, by weight of the composition, provided other milk protein is at least partially derived from the milk protein concentrate.

### The Fatty Acid Material

The fatty acid material utilized in the present invention is selected from fatty acids, non-glyceryl esters thereof, and mixtures thereof. As used herein, the fatty acid material contains a fatty acid chain, or wherein the fatty acid material is a fatty acid ester, contains a fatty acid chain and an ester chain. Thus, wherein the fatty acid material is a fatty acid, the material is depicted as follows:

R-COOH

wherein "R" is the fatty acid chain which is a saturated or unsaturated chain having at least 9 carbon atoms, typically from 9 to 25 carbon atoms, and wherein "COOH" is a carboxylic acid moiety. More preferably, "R" is a saturated or unsaturated chain having from about 11 to about 23, preferably from about 15 to about 21 carbon atoms and, depending upon the embodiment herein, often preferably from about 15 to about 17 carbon atoms. Also preferably, the fatty acid chain contains from 0 to 3 double bonds. Most preferably, the fatty acid chain is unsaturated, in particular having one or two double bonds.

Wherein the fatty acid material is a non-glyceryl ester of a fatty acid (*i*.*e*., a "non-glyceryl ester thereof"), the material is depicted as follows:

R-COOR'

Wherein R is the fatty acid chain as defined above, and R' is the ester chain, with the carboxylate moiety "COO" linking the two together. The ester chain is a straight or branched chain of carbon atoms which is hydrolyzable in the presence of mammalian digestive enzymes, preferably human digestive enzymes, and typically contains no more than about 8 carbon atoms. The ester chain more preferably contains from 1 to about 5 carbon atoms and, again, may be a straight (for example, *n-*propyl) or branched (for example, *iso*-propyl) chain. Highly preferred ester chains include those which form methyl esters (*i*.*e*., R' is -CH₃), ethyl esters, *n*-propyl esters, *iso*-propyl esters, *n*-butyl esters, *iso*-butyl esters, and mixtures thereof. Those ester chains which form ethyl esters are particularly preferred.

In a preferred embodiment of the present invention, the fatty acid material is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomogamma-linolenic acid, eleostearic acid, licanic acid, arachidonic acid, arachidic acid, eicosenoic acid, eicosapentaenoic acid, behenic acid, erucic acid, docosahexaenoic acid, lignoceric acid, non-glyceryl esters thereof, and mixtures thereof. Preferred non-glyceryl esters of fatty acids include ethyl oleate, ethyl linoleate, and mixtures thereof.

In a particularly preferred embodiment of the present invention, the fatty acid material is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomogamma-linolenic acid, eleostearic acid, licanic acid, arachidonic acid, arachidic acid, eicosenoic acid, behenic acid, erucic acid, lignoceric acid, non-glyceryl esters thereof, and mixtures thereof. In this embodiment of the invention, it is particularly preferred to select a fatty acid material containing from 0 to about 3 double bonds and having a fatty acid chain length of from about 15 to about 17 carbon atoms. Additionally, particularly preferred fatty acid materials include oleic acid, linoleic acid, esters thereof, and mixtures thereof. Preferred non-glyceryl esters of this embodiment include ethyl oleate, ethyl linoleate, and mixtures thereof. As an example, ethyl oleate may be obtained from a variety of sources, including Victorian Chemical Co., Richmond, Victoria Australia; Penta Manufacturing Co., Livingston, NJ; and Croda, Inc., Parsippany, NJ.

In another preferred embodiment of the present invention, the fatty acid material is selected from omega-3-fatty acids, non-glyceryl esters thereof, and mixtures thereof. The omega-3-fatty acids are particularly preferred for use herein due to their beneficial effects on the health of the consumer, particularly in the fields of skin and cardiac health.

As is well-understood in the art, and as consistently used herein, the term "omega-3-fatty acid" is utilized to refer to those fatty acid materials having an omega-3 double bond wherein the omega-3 double bond is positioned between the third and fourth carbon atoms of the fatty acid chain when counting from the omega (distal) carbon atom of the chain. Omega-3-fatty acids are preferably derived from marine (fish) sources, including menhaden (a herring-like fish). Non-limiting examples of preferred omega-3-fatty acid sources include OMEGAPURE, commercially available from Omega Protein, Inc., Houston, TX.

Non-limiting examples of omega-3-fatty acids which are suitable for use herein include eicosapentaenoic acid (also known as EPA), docosahexaenoic acid (also known as DHA), and mixtures thereof. Non-glyceryl esters thereof are also contemplated.

In a typical embodiment of the present invention, the compositions comprise from about 0.0001% to about 10% of the fatty acid material, by weight of the composition, and depending upon the particular embodiment desired (for example, a concentrate suitable for further dilution or a ready-to-drink beverage composition). More preferably, the compositions comprise from about 0.01% to about 5% of the fatty acid material, by weight of the composition. Even more preferably, the compositions comprise from about 0.01% to about 3% of the fatty acid material, by weight of the composition. Most preferably, the compositions comprise from about 0.5% to about 2.5% of the fatty acid material, by weight of the composition. For example, a typical composition of the present invention may comprise from about 1% to about 1.3% of the fatty acid material, by weight of the composition.

### Optional Components and Uses of the Present Compositions

The compositions described herein are useful in a wide variety of finished compositions. The compositions are useful as, for example, health care (including pharmaceutical and over-the-counter compositions), food, and beverage compositions, preferably food and beverage compositions, most preferably beverage compositions. Such food and beverage compositions include not only "traditional" foods and beverages, but also those such as dietary supplements and medical foods, and the like, under regulatory guidelines.

The compositions of the present invention may comprise additional optional components to, for example, enhance their performance or to otherwise render the composition more suitable for use as an industrial or consumer product. Such optional components may be dispersed, emulsified, solubilized, or otherwise combined with the fatty acid material and the milk protein concentrate to form the compositions. These components may be added to the compositions herein provided they do not substantially hinder important properties, particularly stabilization and organoleptic properties, of the compositions. Non-limiting examples of optional components are given below:

### Water

Water is typically included in the compositions of the present invention, particularly wherein the compositions are beverage compositions. As used herein, the term "water" includes the total amount of water present in the composition including from, for example, milk protein concentrate or other milk protein source, fruit juice, or vegetable juice, as well as any added water. Water is preferably included at levels from about 10% to about 99.999%, more preferably from about 5% to about 99%, still more preferably at least about 50%, even more preferably at least about 70%, and most preferably from about 70% to about 99%, by weight of the composition. Ready-to-drink beverage compositions will typically comprise at least about 70% water, preferably from about 75% to about 99% water, all by weight of the composition.

### Emulsifiers

It has been found that optional emulsifiers may not interfere with the benefits of the milk protein concentrate herein. Accordingly, the present compositions may optionally comprise from about 0.01% to about 5%, preferably from about 0.02% to about 3%, and most preferably from about 0.04% to about 2%, of an emulsifier, by weight of the composition.

For cloud emulsions, the clouding agent can comprise one or more fats or oils stabilized as an oil-in-water emulsion using a suitable food grade emulsifier. Any of a variety of fats or oils may be employed as the clouding agent, provided that the fat or oil is suitable for use in foods and / or beverages. Preferred are those fats and oils that have been refined, bleached and deodorized to remove off-flavors. Especially suitable for use as clouding agents are those fats that are organoleptically neutral. These include fats from the following sources: vegetable fats such as soybean, corn, safflower, sunflower, cottonseed, canola, and rapeseed; nut fats such as coconut, palm, and palm kernel; and synthetic fats. See e.g., Kupper et al., U.S. Patent No. 4,705,691, issued November 10, 1987, for suitable fat or oil clouding agents.

Any suitable food grade emulsifier can be used that can stabilize the fat or oil clouding agent as an oil-in-water emulsion. Suitable emulsifiers include lecithin, mono-diglycerides, di-glycerides, gum acacia (gum arabic), modified food starches (*e*.*g*., alkenylsuccinate modified food starches), anionic polymers derived from cellulose (*e*.*g*., carboxymethylcellulose), gum ghatti, modified gum ghatti, xanthan gum, tragacanth gum, guar gum, locust bean gum, pectin, and mixtures thereof. See e.g., Kupper et al., U.S. Patent No. 4,705,691, issued November 10, 1987. Modified starches treated to contain hydrophobic as well as hydrophilic groups, such as those described in Caldwell et al., U.S. Patent No. 2,661,349, are preferred emulsifiers for use as herein. Octenyl succinate (OCS) modified starches such as those described in Marotta et al., U.S. Patent No. 3,455,838 and Barndt et al., U.S. Patent No. 4,460,617 are especially preferred emulsifiers.

The clouding agent can be combined with a weighting agent to provide a beverage opacifier that imparts a total or partial opaque effect to the beverage without separating out and rising to the top. The beverage opacifier provides the appearance to the consumer of a juice-containing beverage. Any suitable weighting oil can be employed in the beverage opacifier. Typical weighting oils include brominated vegetable oil, glycerol ester of wood rosin (ester gum), sucrose acetate isobutyrate (SAIB) and other sucrose esters, gum damar, colophony, gum elemi, or others known to those skilled in the art. Other suitable weighting agents include brominated liquid polyol polyesters which are nondigestible. See e.g., Brand et al., U.S. Patent 4,705,690, issued November 10, 1987.

The cloud/opacifier emulsion may be prepared by mixing the clouding agent with the weighting agent (for opacifier emulsions), the emulsifier and water. The emulsion typically contains from about 0% to about 25% clouding agent, from about 0% to about 20% weighting oil agent (in the case of opacifier emulsions), from about 0% to about 30% emulsifiers, and from about 25% to about 97.9% water (or *quantum satis*).

Flavor emulsions useful in beverage compositions of the present invention comprise one or more suitable flavor oils, extracts, oleoresins, essential oils and the like, known in the art for use as flavorants in beverages. This component can also comprise flavor concentrates such as those derived from concentration of natural products such as fruits. Terpeneless citrus oils and essences can also be used herein. Examples of suitable flavors include, for example, fruit flavors such as orange, lemon, lime and the like, cola flavors, tea flavors, coffee flavors, chocolate flavors, dairy flavors. These flavors can be derived from natural sources such as essential oils and extracts, or can be synthetically prepared. The flavor emulsion typically comprises a blend of various flavors and can be employed in the form of an emulsion, alcoholic extract, or spray dried. The flavor emulsion can also include clouding agents, with or without weighting agents, as previously described. See e.g., Kupper et al., U.S. Patent 4,705,691, issued November 10, 1987.

### Flavor Agents

The compositions herein may optionally, but preferably, comprise one or more flavor agents. Preferably, such flavor agents are included in the beverage compositions and are typically selected from fruit juice, fruit flavors, botanical flavors, and mixtures thereof. Wherein fruit juice is included, the beverages of the present invention can comprise from about 0.1% to about 99%, preferably from about 1% to about 50%, more preferably from about 2% to about 30%, and most preferably from about 5% to about 20%, fruit juice. As measured herein, the weight percentage of fruit juice is based on a single strength 2° to 16° Brix fruit juice. The fruit juice can be incorporated into the beverage as a puree, comminute, or as a single strength or concentrated juice. Especially preferred is incorporation of the fruit juice as a concentrate with a solids content (primarily as sugar solids) of from about 20° to about 80° Brix.

Optionally, due to the milk protein of the present compositions, the processes such as those described in Yang et al., U.S. Patent No. 5,648,112, issued July 15, 1997 may be utilized to prepare those compositions containing juice.

The fruit juice can be any citrus juice, non-citrus juice, or mixture thereof, which are known for use in dilute juice beverages. The juice can be derived from, for example, apple, cranberry, pear, peach, plum, apricot, nectarine, grape, cherry, currant, raspberry, gooseberry, elderberry, blackberry, blueberry, strawberry, lemon, lime, mandarin, orange, grapefruit, cupuacu, potato, tomato, lettuce, celery, spinach, cabbage, watercress, dandelion, rhubarb, carrot, beet, cucumber, pineapple, coconut, pomegranate, kiwi, mango, papaya, banana, watermelon, passion fruit, tangerine, and cantaloupe. Preferred juices are derived from apple, pear, lemon, lime, mandarin, grapefruit, cranberry, orange, strawberry, tangerine, grape, kiwi, pineapple, passion fruit, mango, guava, raspberry and cherry. Citrus juices, preferably grapefruit, orange, lemon, lime, and mandarin juices, as well as juices derived from mango, apple, passion fruit, and guava, as well as mixtures of these juices are most preferred.

Fruit flavors may also be utilized. As described above with respect to flavor emulsions, fruit flavors may be derived from natural sources such as essential oil and extracts, or can be synthetically prepared. Fruit flavors may be derived from fruits through processing, particularly concentrating. Wherein fruit juices are concentrated or evaporated, the water which is removed or the condensate contains volatile substances which comprise the flavor of the fruit. Often, such flavor is added to a juice concentrate to enhance the flavor thereof. The condensate may also be used to flavor "near waters" (lightly flavored water).

Botanical flavors may also be utilized. As used herein, the term "botanical flavor" refers to a flavor derived from parts of a plant other than the fruit; i.e., derived from nuts, bark, roots, and / or leaves. Also included within the term "botanical flavor" are synthetically prepared flavors made to simulate botanical flavors derived from natural sources. Botanical flavors can be derived from natural sources such as essential oils and extracts, or can be synthetically prepared. Suitable botanical flavors include chocolate, vanilla, jamaica, kola, marigold, chrysanthemum, chamomile, ginger, valerian, yohimbe, hops, eriodictyon, ginseng, bilberry, rice, red wine, mango, peony, lemon balm, nut gall, oak chip, lavender, walnut, gentiam, luo han guo, cinnamon, angelica, aloe, agrimony, yarrow and mixtures thereof. Particularly preferred flavors include chocolate or vanilla.

Wherein coffee solids are included, the compositions typically comprise from about 3% to about 23% coffee solids, by weight of the composition. Wherein tea solids are included, the compositions of the present invention can comprise from about 0.01% to about 1.2%, preferably from about 0.05% to about 0.8%, by weight of the composition, of tea solids. The term "tea solids" as used herein means solids extracted from tea materials including those materials obtained from the genus *Camellia* including *C. sinensis* and *C. assaimica*, for instance, freshly gathered tea leaves, fresh green tea leaves that are dried immediately after gathering, fresh green tea leaves that have been heat treated before drying to inactivate any enzymes present, unfermented tea, instant green tea, and partially fermented tea leaves. Green tea solids are tea leaves, tea plant stems, and other plant materials that are related and which have not undergone substantial fermentation to create black teas. Members of the genus *Phyllanthus, Catechu gambir* and Uncaria family of tea plants can also be used. Mixtures of unfermented and partially fermented teas can be used.

### Sweeteners

The compositions of the present invention can, and typically will, contain an effective amount of one or more sweeteners, including carbohydrate sweeteners and natural or artificial no/low calorie sweeteners. The amount of the sweetener used in the compositions of the present invention typically depends upon the particular sweetener used and the sweetness intensity desired. For no/low calorie sweeteners, this amount varies depending upon the sweetness intensity of the particular sweetener.

The compositions of the present invention can be sweetened with any of the carbohydrate sweeteners, preferably monosaccharides and / or disaccharides. Sweetened compositions, particularly beverages, will typically comprise from about 0.1% to about 40%, more preferably from about 0.1% to about 20%, and most preferably from about 6% to about 14%, sweetener, all by weight of the composition. These sweeteners can be incorporated into the compositions in solid or liquid form (such as a syrup).

Preferred sugar sweeteners for use in compositions of the present invention are sucrose, fructose, glucose, and mixtures thereof. Fructose can be obtained or provided as liquid fructose, high fructose corn syrup, dry fructose or fructose syrup. Other naturally occurring sweeteners or their purified extracts, such as glycyrrhizin, the protein sweetener thaumatin, the juice of Luo Han Guo disclosed in, for example, Fischer et al., U.S. Patent No. 5,433,965, issued July 18, 1995, and the like can also be used in the compositions of the present invention.

Suitable no/low calorie sweeteners include, for example, saccharin, cyclamates, L-aspartyl-L-phenylalanine lower alkyl ester sweeteners (*e*.*g*., aspartame); L-aspartyl-D-alanine amides disclosed in Brennan et al., U.S. Patent No. 4,411,925; L-aspartyl-D-serine amides disclosed in Brennan et al., U.S. Patent 4,399,163; L-aspartyl-L-1-hydroxymethylalkaneamide sweeteners disclosed in Brand, U.S. Patent No. 4,338,346; L-aspartyl-1-hydroxyethyalkaneamide sweeteners disclosed in Rizzi, U.S. Patent No. 4,423,029; L-aspartyl-D-phenylglycine ester and amide sweeteners disclosed in Janusz, European Patent Application 168,112, published January 15, 1986; N-[N-3,3-dimethylbutyl)-L-alpha-aspartyl]-L-phenylalanine 1-methyl ester sweeteners disclosed in Gerlat et al., WO 99/30576, assigned to The Nutrasweet Co., published June 24, 1999; alltame, thaumatin; dihydrochalcones; cyclamates; steviosides; glycyrrhizins, synthetic alkoxy aromatics, such as Dulcin and P-4000; sucralose; suosan; miraculin; monellin; sorbitol, xylitol; talin; cyclohexylsulfamates; substituted imidazolines; synthetic sulfamic acids such as acesulfame, acesulfame-K and n-substituted sulfamic acids; oximes such as perilartine; peptides such as aspartyl malonates and succanilic acids; dipeptides; amino acid based sweeteners such as gem-diaminoalkanes, *meta*-aminobenzoic acid, L-aminodicarboxylic acid alkanes, and amides of certain alpha-aminodicarboxylic acids and gemdiamines; and 3-hydroxy-4-alkyloxyphenyl aliphatic carboxylates or heterocyclic aromatic carboxylates; erythritol; and mixtures thereof.

### Coloring Agent

Coloring agents may be utilized in the compositions of the present invention. For example, natural or artificial colors may be used.

FD&C dyes (*e*.*g*., yellow #5, blue #2, red # 40) and / or FD&C lakes are preferably used. By adding the lakes to the other powdered ingredients, all the particles, in particular the colored iron compound, are completely and uniformly colored and a uniformly colored composition is attained. Preferred lake dyes which may be used in the present invention are the FDA-approved Lake, such as Lake red #40, yellow #6, blue #1, and the like. Additionally, a mixture of FD&C dyes or a FD&C lake dye in combination with other conventional food and food colorants may be used.

Other coloring agents, for example, natural agents may be utilized. Non-limiting examples of such other coloring agents include fruit and vegetable juices, riboflavin, carotenoids (for example, beta-carotene), tumeric, and lycopenes.

The exact amount of coloring agent used will vary, depending on the agents used and the intensity desired in the finished composition. Generally, if utilized, the coloring agent is typically present at a level of from about 0.0001% to about 0.5%, preferably from about 0.001% to about 0.1%, and most preferably from about 0.004% to about 0.1 %, by weight of the composition.

### Nutrients

The compositions herein can be fortified with one or more nutrients, defined herein as one or more vitamins and / or minerals. The U.S. Recommended Daily Intake (USRDI) for vitamins and minerals are defined and set forth in the Recommended Daily Dietary Allowance-Food and Nutrition Board, National Academy of Sciences-National Research Council.

Unless otherwise specified herein, wherein a given mineral is present in the composition, the composition comprises at least about 1%, preferably at least about 5%, more preferably from about 10% to about 200%, even more preferably from about 40% to about 150%, and most preferably from about 60% to about 125% of the USRDI of such mineral. Unless otherwise specified herein, wherein a given vitamin is present in the composition, the composition comprises at least about 1%, preferably at least about 5%, more preferably from about 10% to about 200%, even more preferably from about 20% to about 150%, and most preferably from about 25% to about 120% of the USRDI of such vitamin.

Commercially available vitamin A sources may also be included in the present compositions. As used herein, "vitamin A" includes, but is not limited to, retinol, β-carotene, retinol palmitate, and retinol acetate. The vitamin A may be in the form of, for example, an oil, beadlets or encapsulated.

Wherein vitamin A is present in the compositions herein, the composition comprises at least about 1%, preferably at least about 5%, more preferably from about 10% to about 200%, even more preferably from about 15% to about 150%, and most preferably from about 20% to about 120% of the USRDI of vitamin A. The quantity of vitamin A to be added is dependent on processing conditions and the amount of vitamin A deliver desired after storage. Preferably, wherein vitamin A is included within the present compositions, the compositions comprise from about 0.0001% to about 0.2%, more preferably from about 0.0002% to about 0.12%, also preferably from about 0.0003% to about 0.1%, even more preferably from about 0.0005% to about 0.08%, and most preferably from about 0.001% to about 0.06% of vitamin A, by weight of the composition.

Commercially available sources of vitamin B₂ (also known as riboflavin) may be utilized in the present compositions. Wherein vitamin B₂ is present in the compositions herein, the composition comprises at least about 1%, preferably at least about 5%, more preferably from about 5% to about 200%, even more preferably from about 10% to about 100%, and most preferably from about 10% to about 50% of the USRDI of vitamin B₂.

Commercially available sources of vitamin C can be used herein. Encapsulated ascorbic acid and edible salts of ascorbic acid can also be used. Wherein vitamin C is present in the compositions herein, the composition comprises at least about 1%, preferably at least about 5%, more preferably from about 10% to about 200%, even more preferably from about 20% to about 150%, and most preferably from about 25% to about 120% of the USRDI of such vitamin.

The quantity of vitamin C to be added is dependent on processing conditions and the amount of vitamin C deliver desired after storage. Preferably, wherein vitamin C is included within the present compositions, the compositions comprise from about 0.005% to about 0.2%, more preferably from about 0.01% to about 0.12%, also preferably from about 0.02% to about 0.1%, even more preferably from about 0.02% to about 0.08%, and most preferably from about 0.03% to about 0.06% of vitamin C, by weight of the composition.

Nutritionally supplemental amounts of other vitamins which may be incorporated herein include, but are not limited to, biotin, vitamins B₁, B₃, B₆ and B₁₂, folic acid, pantothenic acid, folic acid, vitamin D, and vitamin E. Wherein the composition comprises one of these vitamins, the composition preferably comprises at least 5%, preferably at least 25%, and most preferably at least 35% of the USRDI for such vitamin.

Non-limiting examples of minerals include iodine, chromium, magnesium, manganese, molybdenum, selenium, phosphorous, magnesium, zinc, iodine, iron, and copper. As an example, any soluble salt of these minerals suitable for inclusion in edible compositions can be used, for example, magnesium citrate, magnesium gluconate, magnesium sulfate, zinc chloride, zinc sulfate, potassium iodide, copper sulfate, copper gluconate, and copper citrate.

Calcium is a particularly preferred mineral for use in the present invention. Preferred sources of calcium include, for example, amino acid chelated calcium, calcium carbonate, calcium oxide, calcium hydroxide, calcium sulfate, calcium chloride, calcium phosphate, calcium hydrogen phosphate, calcium dihydrogen phosphate, calcium citrate, calcium malate, calcium titrate, calcium gluconate, calcium propionate, tricalcium phosphate, and calcium lactate, and in particular calcium citrate-malate.

The form of calcium citrate-malate is described in, *e*.*g*., U.S. Patent Nos. 5,670,344; 5,612,026; 5,571,441; 5,474,793; 5,468,506; 5,445,837; 5,424,082; 5,422,128; 5,401,524; 5,389,387; 5,314,919; 5,232,709; 5,225,221; 5,215,769; 5,186,965; 5,151,274; 5,128,374; 5,118,513; 5,108,761; 4,994,283; 4,786,510; and 4,737,375.

Wherein calcium is included, the compositions will typically comprise from about 0.01% to about 0.5%, more preferably from about 0.03% to about 0.2%, even more preferably from about 0.05% to about 0.15%, and most preferably from about 0.1% to about 0.15% of calcium, all by weight of the composition.

Iron may be utilized in the compositions of the present invention. Acceptable forms of iron are well-known in the art. The amount of iron compound incorporated into the composition will vary widely depending upon the level of supplementation desired in the final composition and the targeted consumer. Iron fortified compositions of the present invention typically contain from about 5% to about 100%, preferably from about 15% to about 50%, and most preferably about 20% to about 40% of the USRDI for iron.

Highly bioavailable ferrous salts that can be used in the ingestible compositions of the present invention are ferrous sulfate, ferrous fumarate, ferrous succinate, ferrous gluconate, ferrous lactate, ferrous tartarate, ferrous citrate, ferrous amino acid chelates, as well as mixtures of these ferrous salts. While ferrous iron is typically more bioavailable, certain ferric salts can also provide highly bioavailable sources of iron.

Certain ferric salts can also provide highly bioavailable sources of iron. Highly bioavailable ferric salts that can be used in the food or beverage compositions of the present invention are ferric saccharate, ferric ammonium citrate, ferric citrate, ferric sulfate, ferric pyrophosphate, ferric orthophosphate, as well as mixtures of these ferric salts. Combinations or mixtures of highly bioavailable ferrous and ferric salts can be used.

A particularly preferred ferric iron source is ferric pyrophosphate, for example, microencapsulated SUNACTIVE Iron, commercially available from Taiyo International, Inc., Edina, Minnesota, U.S.A and Yokkaichi, Mie, Japan. SUNACTIVE Iron is particularly preferred for use herein due to its particle size, compatibility, and bioavailability.

Ferrous amino acid chelates particularly suitable as highly bioavailable iron sources for use in the present invention are those having a ligand to metal ratio of at least 2: 1. For example, suitable ferrous amino acid chelates having a ligand to metal mole ratio of two are those of formula:

Fe(L)₂

where L is an alpha amino acid, dipeptide, tripeptide, or quadrapeptide ligand. See e.g., U.S. Patent Nos. 4,863,898; 4,830,716; and 4,599,152. Particularly preferred ferrous amino acid chelates are those where the reacting ligands are glycine, lysine, and leucine. Most preferred is the ferrous amino acid chelate sold under the mark FERROCHEL (Albion Laboratories, Salt Lake City, Utah) wherein the ligand is glycine.

Other sources of iron particularly suitable for fortifying compositions of the present invention included certain iron-sugar-carboxylate complexes. In these iron-sugar-carboxylate complexes, the carboxylate provides the counterion for the ferrous or ferric iron. These iron-sugar-carboxylate complexes can be prepared in the manner described in, *e*.*g*., Nakel et al., U.S. Patent Nos. 4,786,510 and 4,786,518, issued November 22, 1988. These materials are referred to as "complexes", but they may exist in solution as complicated, highly hydrated, protected colloids; the term "complex" is used for the purpose of simplicity.

Zinc may also be utilized in the compositions of the present invention. Acceptable forms of zinc are well-known in the art. Zinc fortified compositions of the present invention typically contain from about 5% to about 100%, preferably from about 15% to about 50%, and most preferably about 25% to about 45% of the USRDI for zinc. The zinc compounds which can be used in the present invention can be in any of the commonly used forms such as, *e*.*g*., zinc sulfate, zinc chloride, zinc acetate, zinc gluconate, zinc ascorbate, zinc citrate, zinc aspartate, zinc picolinate, amino acid chelated zinc, and zinc oxide. Zinc oxide is particularly preferred.

### Edible Carriers

One or more edible carriers may be utilized in the present compositions. In particular, for example wherein one or more nutrients is included within the compositions, the edible carrier comprises an emulsifier such as that described in Mehansho et al., U.S. Patent No. 5,888,563, issued March 30, 1999. Such edible carriers may reduce undesirable flavors and colors associated with such nutrients. A preferred edible carrier for use herein is lecithin. Lecithin may be commercially obtained, for example, as soy lecithin (commercially available from Central Soya, Fort Wayne, IN).

### Fiber

Fibers are well-known in the art and include complex carbohydrates resistant to digestion by mammalian enzymes, such as the carbohydrates found in plant cell walls and seaweed, and those produced by microbial fermentation. Examples of these complex carbohydrates are brans, celluloses, hemicelluloses, pectins, gums and mucilages, seaweed extract, and biosynthetic gums. Sources of the cellulosic fiber include vegetables, fruits, seeds, cereals, and man-made fibers (for example, by bacterial synthesis). Commercial fibers such as purified plant cellulose, or cellulose flour, can also be used. Naturally occurring fibers include fiber from whole citrus peel, citrus albedo, sugar beets, citrus pulp and vesicle solids, apples, apricots, and watermelon rinds.

Particularly preferred fibers for use herein are glucose polymers, preferably those which have branched chains, and which are typically less digestible relative to starches and maltodextrins. Preferred among these fibers is one marketed under the trade name FIBERSOL2, commercially available from Matsutani Chemical Industry Co., Itami City, Hyogo, Japan.

Fructo-oligosaccharides are also preferred fibers herein. The preferred fructo-oligosaccharides are a mixture of fructo-oligosaccharides composed of a chain of fructose molecules linked to a molecule of sucrose. Most preferably, they have a nystose to kestose to fructosyl-nystose ratio of about 40:50: 10, by weight of the composition. Preferred fructo-oligosaccharides may be obtained by enzymatic action of fructosyltransferase on sucrose such as those which are, for example, commercially available from Beghin-Meiji Industries, Neuilly-sur-Seine, France.

Other preferred fibers for use herein include arabinogalactans. Non-limiting examples of preferred, commercially available sources of arabinogalactan include LAREX UF, LARACARE A200, IMMUNENHANCER (CAS No. 9036-66-2), CLEARTRAC, FIBERAID, and AC-9, all commercially available from (for example) Larex, Inc. of St. Paul, Minnesota, U.S.A.

These dietary fibers may be in a crude or purified form. The dietary fiber used may be of a single type (*e*.*g*., cellulose), a composite dietary fiber (*e*.*g*., citrus albedo fiber containing cellulose and pectin), or some combination of fibers (*e*.*g*., cellulose and a gum). The fibers can be processed by methods known to the art.

Wherein a fiber is utilized, the desired total level of fiber for the present compositions of the present invention is typically from about 0.01% to about 15%, preferably from about 0.1% to about 5%, more preferably from about 0.1% to about 3%, and most preferably from about 0.2% to about 2%, all by weight of the composition. The total amount of fiber includes any added fiber as well as any soluble dietary fiber naturally present in any other component of the present invention.

### Carbonation Component

Carbon dioxide can be introduced into the water which is mixed with a beverage syrup or into the dilute beverage after dilution to achieve carbonation. The carbonated beverage can be placed into a container, such as a bottle or can, and then sealed. Any conventional carbonation methodology may be utilized to make carbonated beverage compositions of this invention. The amount of carbon dioxide introduced into the beverage will depend upon the particular flavor system utilized and the amount of carbonation desired.

### pH

The compositions of the present invention may have various pH levels. For example, the compositions may be acidic in nature (for example a pH of from about 3 to about 5) or more basic. Preferred compositions of the present invention have a pH of from about 6 to about 8, more preferably from about 6.3 to about 7.4.

Organic as well as inorganic edible acids may be used to lower the pH of the beverage composition. The acids can be present in their undissociated form or, alternatively, as their respective salts, for example, potassium or sodium hydrogen phosphate, potassium or sodium dihydrogen phosphate salts. The preferred acids are edible organic acids including citric acid, malic acid, fumaric acid, adipic acid, phosphoric acid, gluconic acid, tartaric acid, ascorbic acid, acetic acid, phosphoric acid or mixtures thereof. The most preferred acids are citric and malic acids. Glucono Delta Lactone (GDL) is also a preferred acid for use herein, particularly wherein it is desired to reduce pH without introducing excessive acidic, or tart, flavor in to the final composition.

For those compositions that are more basic in nature, various bases may be utilized. For example, sodium hydroxide or potassium hydroxide may be used herein.

### Processes of Preparing the Present Compositions

The compositions of the present invention may be prepared by a process comprising combining:
(a) milk protein concentrate; and
(b) a fatty acid material selected from the group consisting of fatty acids, non-glyceryl esters thereof, and mixtures thereof.
consisting of ethyl oleate, oleic acid, and mixtures thereof, then the composition is substantially free.

The compositions are otherwise prepared by conventional methods that will be well-known to one of ordinary skill. To illustrate, the compositions of the present invention may be prepared by dissolving, dispersing, or otherwise mixing all components singularly or in suitable combinations together and in water where appropriate, agitating with a mechanical stirrer until all of the ingredients have been solubilized or adequately dispersed. Where appropriate, all separate solutions and dispersions may then be combined. Wherein a shelf stable composition is desired, the final mixture can optionally, but preferably, be pasteurized, retorted, canned, or filled aseptically under appropriate process conditions.

The compositions may be prepared in accordance with methods known to one of ordinary skill in the art. Otherwise, if desired, the following process may be consulted for preparation of the present compositions:

Water is added to a high shear mixing apparatus and agitation is commenced (optionally, the apparatus may be in recirculation with, for example, a mixing tank to enable preparation of large batch sizes). The agitation may be a mixing energy of, for example, from about 5 Watt/Kg (watts per kilogram) to about 55 W/kg. A specific order of addition of the various components of the composition may not be critical to achieve the stabilizing and flavor masking benefits of the present compositions. After the addition of carrageenans or other like materials (if used), the batch is allowed to mix for at least about five minutes to allow proper hydration. Otherwise, all components may be added sequentially with little or no waiting period between the addition of each component. Once the various components are added, the composition may optionally be homogenized at a pressure of from about 3,000 psi to about 7,000 psi. The composition may optionally be sterilized under ultra-high temperature (UHT) conditions. The composition may optionally be homogenized at a pressure of from about 2,000 psi to about 5,000 psi. Typically, homogenization occurs either prior or subsequent to sterilization, but homogenization may occur both prior and subsequent to homogenization. The composition may be optionally aseptically filled into an appropriate aseptic containing device.

The means for subjecting the components to the mixing energy may be selected from a variety of well-known apparatuses (energizing means). For example, this energizing means may be a mixer which provides energy to the liquid medium by forming ultrasonic vibrations therein, *e*.*g*., a Sonolator, commercially available from Sonic Corporation, Stratford, CT or Piezoelectric transducers. The Sonolator is an in-line system providing ultrasonic vibrations by pumping a liquid, a blend of liquids, or a solid dispersion in a liquid through a shaped orifice at a high linear velocity. The liquid stream impinges against a blade cantilevered in the stream. Flow over the blade causes vibrations in the blade that produces cavitation in the stream converting flow energy into mixing/dispersion energy. Other particularly useful energizing means include batch mixers providing a high agitator tip speed, *e*.*g*., blenders as available from Sunbeam Corporation of Delray Beach, FL with the brand name OSTERIZER. Additionally rotor/stator high shear mixers, commercially available from Charles Ross & Son, Hauppauge, NY may be useful. In-line mixers such as are available from Quadro Inc., Millburn, NJ, as model Quadro ZC/XC are useful as well. Additionally, particularly preferred energizing means for use herein include bottom-driven mixtures such as the Breddo Likwifier (Model LOR, round tank; Model LTD square tank) commercially available from Breddo Likwifier, Kansas City, MO and APV Mixer/Blender (Multiverter (round tank) / Liquiverter (square tank) high speed mixers commercially available from APV Crepaco, Inc., Lake Mills, WI.

### EXAMPLES

The following are non-limiting examples of the present compositions. The following examples are provided to illustrate the invention and are not intended to limit the scope thereof in any manner.

### Example 1

A vanilla flavored beverage composition is prepared having the following components in approximately the indicated amounts:

| **Component** | **Weight Percent** |
|---|---|
| Carrageenan (commercially available from FMC Biopolymer Food & Specialty Business, Philadelphia, PA) | 0.04 |
| Cellulose Gum (commercially available from FMC Biopolymer Food & Specialty Business, Philadelphia, PA) | 0.52 |
| Gum Arabic (commercially available from TIC Gums, Inc., Belcamp, MD) | 0.41 |
| Nutrients | 0.45 |
| Soy Lecithin (commercially available from Central Soya, Fort Wayne, IN) | 0.03 |
| Ethyl Oleate (commercially available from Victorian Chemical Co., Richmond, Victoria Austrailia) | 1.08 |
| Sodium Caseinate | 0.3 |
| Milk Protein Concentrate (80% milk protein) (commercially available from New Zealand Milk Products, Lemoyne, PA) | 3 |
| Sugar | 4 |
| Sucralose Liquid Concentrate (commercially available from McNeil | 0.03 |
| Specialty, McIntosh, AL) | |
| Flavoring Agents, including vanilla flavor | 0.75 |
| Water | *Quantum satis* |

### Example 2

Eleven fluid ounces of a chocolate flavored beverage composition containing approximately 4 grams of ethyl oleate is prepared having the following components in approximately the indicated amounts:

| **Component** | **Weight Percent** |
|---|---|
| Sodium Hydroxide | 0.03 |
| Carrageenan (commercially available from FMC Biopolymer Food & Specialty Business, Philadelphia, PA) | 0.05 |
| Cellulose Gum (commercially available from FMC Biopolymer Food & Specialty Business, Philadelphia, PA) | 0.44 |
| Gum Arabic (commercially available from TIC Gums, Inc., Belcamp, MD) | 0.45 |
| Starch (commercially available from National Starch & Chemical, Bridgewater, NJ) | 1.5 |
| Nutrients | 0.5 |
| Emulsifier | 0.04 |
| Ethyl Oleate (commercially available from Victorian Chemical Co., Richmond, Victoria Austrailia) | 1.18 |
| Sodium Caseinate | 0.33 |
| Milk Protein Concentrate (80% milk protein) (commercially available from New Zealand Milk Products, Lemoyne, PA) | 3.23 |
| Sugar | 4 |
| Maltodextrin | 2 |
| Sucralose Liquid Concentrate (commercially available from McNeil Specialty, McIntosh, AL) | 0.06 |
| Flavoring Agents, including cocoa | 2.75 |
| Water | *Quantum satis* |

### Example 3

Eleven fluid ounces of a chocolate flavored beverage composition containing approximately 4 grams of ethyl oleate, 10 grams of fiber, and 14 grams of protein is prepared having the following components in approximately the indicated amounts:

| **Component** | **Weight Percent** |
|---|---|
| Sodium Polyphosphate | 0.01 |
| Sodium Hydroxide | 0.03 |
| Carrageenan (commercially available from FMC Biopolymer Food & Specialty Business, Philadelphia, PA) | 0.1 |
| Cellulose Gum (commercially available from FMC Biopolymer Food & Specialty Business, Philadelphia, PA) | 0.44 |
| Gum Arabic (commercially available from TIC Gums, Inc., Belcamp, MD) | 2.17 |
| Nutrients, including Calcium Propionate | 0.74 |
| Emulsifier | 0.04 |
| Ethyl Oleate (commercially available from Victorian Chemical Co., Richmond, Victoria Austrailia) | 1.18 |
| Flavoring Agents, including cocoa | 1.75 |
| Silicone Emulsion | 0.002 |
| Sodium Caseinate | 0.33 |
| Milk Protein Concentrate (80% milk protein) (commercially available from New Zealand Milk Products, Lemoyne, PA) | 4.7 |
| White Sugar | 4 |
| Maltodextrin | 3 |
| Sucralose Liquid Concentrate (commercially available from McNeil Specialty, McIntosh, AL) | 0.06 |
| Water | *Quantum satis* |

### Example 4

Eleven fluid ounces of a chocolate flavored beverage composition containing approximately 4 grams of ethyl oleate and 10 grams of protein is prepared having the following components in the indicated amounts:

| **Component** | **Weight Percent** |
|---|---|
| Carrageenan (commercially available from FMC Biopolymer Food & Specialty Business, Philadelphia, PA) | 0.06 |
| Cellulose Gum (commercially available from FMC Biopolymer Food & Specialty Business, Philadelphia, PA) | 0.49 |
| Gum Arabic (commercially available from TIC Gums, Inc., Belcamp, MD) | 0.45 |
| Nutrients, including calcium propionate and ferric orthophosphate | 0.65 |
| Emulsifier | 0.04 |
| Ethyl Oleate (commercially available from Victorian Chemical Co., Richmond, Victoria Austrailia) | 1.18 |
| Sodium Caseinate | 0.33 |
| Milk Protein Concentrate (80% milk protein) (commercially available from New Zealand Milk Products, Lemoyne, PA) | 1.43 |
| Nonfat Dry Milk Powder (commercially available from O-AT-KA Milk Products Cooperative, Inc., Batavia, KY) | 4.23 |
| White Sugar | 4 |
| Maltodextrin | 3 |
| Sucralose Liquid Concentrate (commercially available from McNeil Specialty, McIntosh, AL) | 0.06 |
| Flavoring Agents, including cocoa | 1.7 |
| Water | *Quantum satis* |

### Example 5

Eleven fluid ounces of a chocolate flavored beverage composition containing approximately 4 grams of ethyl oleate and 10 grams of protein is prepared having the following components in approximately the indicated amounts:

| **Component** | **Weight Percent** |
|---|---|
| Sodium Polyphosphate | 0.01 |
| Sodium Hydroxide | 0.03 |
| Carrageenan (commercially available from FMC Biopolymer Food & Specialty Business, Philadelphia, PA) | 0.06 |
| Cellulose Gum (commercially available from FMC Biopolymer Food & Specialty Business, Philadelphia, PA) | 0.44 |
| Gum Arabic (commercially available from TIC Gums, Inc., Belcamp, MD) | 0.45 |
| Nutrients, including calcium propionate and ferric orthophosphate) | 0.65 |
| Emulsifier | 0.04 |
| Ethyl Oleate (commercially available from Victorian Chemical Co., Richmond, Victoria Austrailia) | 1.18 |
| Sodium Caseinate | 0.33 |
| Milk Protein Concentrate (80% milk protein) (commercially available from New Zealand Milk Products, Lemoyne, PA) | 2.31 |
| Nonfat Dry Milk Powder (commercially available from O-AT-KA Milk Products Cooperative, Inc., Batavia, KY) | 2.14 |
| White Sugar | 4 |
| Maltodextrin | 3 |
| Sucralose Liquid Concentrate (commercially available from McNeil Specialty, McIntosh, AL) | 0.06 |
| Flavoring Agents, including cocoa | 1.7 |
| Water | *Quantum satis* |

## Claims

1. A composition comprising:
a) from 0.5% to 4% milk protein concentrate, wherein the milk protein concentrate comprises at least 65% milk protein;
b) less than 6% dry milk; and
c) a fatty acid material selected from fatty acids, non-glyceryl esters thereof, and mixtures thereof;
wherein, when the composition comprises less than 0.02% ethyl oleate, oleic acid, and mixtures thereof, the composition also comprises less than 1% lactose from sources other than the milk protein concentrate.

2. The composition according to Claim 1 wherein the fatty acid chain of the fatty acid material contains from 0 to 3 double bonds and has a length of from 9 to 25 carbon atoms.

3. The composition according to any of the preceding claims wherein the composition comprises less than 1% lactose.

4. The composition according to any of the preceding claims comprising from 0.5% to 2.5% of the fatty acid material, by weight of the composition.

5. The composition according to any of the preceding claims which is a ready-to-drink beverage composition comprising at least 70% water, by weight of the composition.

6. The composition according to any of the preceding claims comprising from 1% to 3.5% milk protein concentrate, by weight of the composition.

7. The composition according to any of the preceding claims wherein the milk protein concentrate comprises at least 75% milk protein, by weight of the milk protein concentrate.

8. The composition according to any of the preceding claims wherein the fatty acid material is selected from oleic acid, linoleic acid, non-glyceryl esters thereof, and mixtures thereof.

9. The composition according to any of the preceding claims further comprising from 0.1% to 3% fiber.

## Patentansprüche

1. Zusammensetzung, umfassend:
a) zu 0,5 % bis 4 % Milchproteinkonzentrat, wobei das Milchproteinkonzentrat zu mindestens 65 % Milchprotein umfasst;
b) zu weniger als 6 % Trockenmilch; und
c) ein Fettsäurematerial, ausgewählt aus Fettsäuren, Nicht-Glycerylestern davon und Mischungen davon;
wobei, wenn die Zusammensetzung zu weniger als 0,02 % Ethyloleat, Oleinsäure und Mischungen davon umfasst, die Zusammensetzung auch zu weniger als 1 % Lactose aus anderen Quellen als dem Milchproteinkonzentrat umfasst.

2. Zusammensetzung nach Anspruch 1, wobei die Fettsäurekette des Fettsäurematerials 0 bis 3 Doppelbindungen enthält und eine Länge von 9 bis 25 Kohlenstoffatomen aufweist.

3. Zusammensetzung nach einem der vorstehenden Ansprüche, wobei die Zusammensetzung zu weniger als 1 % Lactose umfasst.

4. Zusammensetzung nach einem der vorstehenden Ansprüche, die zu 0,5 Gew.-% bis 2,5 Gew.-% der Zusammensetzung Fettsäurematerial umfasst.

5. Zusammensetzung nach einem der vorstehenden Ansprüche, die eine trinkfertige Getränkezusammensetzung ist, die zu mindestens 70 %, bezogen auf das Gewicht der Zusammensetzung, Wasser umfasst.

6. Zusammensetzung nach einem der vorstehenden Ansprüche, die zu 1 Gew.-% bis 3,5 Gew.-% der Zusammensetzung Milchproteinkonzentrat umfasst.

7. Zusammensetzung nach einem der vorstehenden Ansprüche, wobei das Milchproteinkonzentrat zu mindestens 75 %, bezogen auf das Gewicht des Milchproteinkonzentrats, Milchprotein umfasst.

8. Zusammensetzung nach einem der vorstehenden Ansprüche, wobei das Fettsäurematerial aus Oleinsäure, Linolsäure, Nicht-Glycerylestern davon und Mischungen davon ausgewählt ist.

9. Zusammensetzung nach einem der vorstehenden Ansprüche, die ferner zu 0,1 % bis 3 % Fasern umfasst.

## Revendications

1. Composition comprenant :
a) de 0,5 % à 4 % de concentrat de protéines de lait, où le concentrat de protéines de lait comprend au moins 65 % de protéines de lait ;
b) moins de 6 % de lait déshydraté ; et
c) une matière de type acide gras choisie parmi les acides gras, les esters non glycéryliques de ceux-ci et leurs mélanges ;
où, lorsque la composition comprend moins de 0,02 % d'oléate d'éthyle, d'acide oléique, et leurs mélanges, la composition comprend également moins de 1 % de lactose provenant de sources autres que le concentrat de protéines de lait.

2. Composition selon la revendication 1, dans laquelle la chaîne d'acide gras de la matière de type acide gras contient de 0 à 3 doubles liaisons et a une longueur de 9 à 25 atomes de carbone.

3. Composition selon l'une quelconque des revendications précédentes, où la composition comprend moins de 1 % de lactose.

4. Composition selon l'une quelconque des revendications précédentes, comprenant de 0,5 % à 2,5 % de matière de type acide gras, en poids de la composition.

5. Composition selon l'une quelconque des revendications précédentes, qui est une composition de type boisson prête à être consommée, comprenant au moins 70 % d'eau, en poids de la composition.

6. Composition selon l'une quelconque des revendications précédentes, comprenant de 1 % à 3,5 % de concentrat de protéines de lait, en poids de la composition.

7. Composition selon l'une quelconque des revendications précédentes, dans laquelle le concentrat de protéines de lait comprend au moins 75 % de protéines de lait, en poids du concentrat de protéines de lait.

8. Composition selon l'une quelconque des revendications précédentes, dans laquelle la matière de type acide gras est choisie parmi l'acide oléique, l'acide linoléique, les esters non glycéryliques de ceux-ci, et leurs mélanges.

9. Composition selon l'une quelconque des revendications précédentes, comprenant, en outre, de 0,1 % à 3 % de fibres.
